# EUROPEAN PATENT SPECIFICATION

(11) **EP 2 196 157 B1**
(45) Date of publication and mention of the grant of the patent: **13.02.2013**
(21) Application number: 09252752.2
(22) Date of filing: 08.12.2009
(51) Int. Cl.: A61B 17/06

(54) **Suture packaging**
Verpackung für chirurgisches Nahtmaterial
Emballage de suture

(30) Priority: 08.12.2008 US 120627 P; 16.11.2009 US 619008
(43) Date of publication of application: 16.06.2010
(73) Proprietor: Covidien LP, Mansfield, MA 02048 (US)
(72) Inventor: Kirsch, David, Madison CT 06443 (US)
(74) Representative: Soames, Candida Jane

(56) References cited:
- EP-A2- 0 091 792
- EP-A2- 0 749 725
- DE-A1- 2 618 662
- US-A- 4 572 363

## Description

### BACKGROUND

### Technical Field

The present disclosure relates to packaging for sutures. More particularly, the present disclosure relates to suture packages for receiving barbed sutures.

### Background of Related Art

Sutures and packages for retaining sutures are known in the art. Suture packages may be of the foldable type, constructed from paper and configured to form a pocket for receiving one or more sutures therein. Alternatively, the suture packages may be of the molded variety. Such packages typically define a channel for the receipt of one or more sutures therein.

Packaging for barbed sutures is not as well known in the art. Packaging barbed sutures is complicated by the configuration of the sutures. In addition to the normal problems associated with packaging multiple sutures or overlapping lengths of the same suture, namely, entangling of the suture(s), the barbs formed along the length of a barbed suture increase the likelihood that the suture may become entangled with itself or other barbed sutures. When excessive force is applied to the barbs while untangling the suture, damage may result to the barbs formed on the suture, thereby diminishing the effectiveness of the suture.

Therefore, it would be beneficial to have a suture package configured to retain one or more barbed sutures such that the suture does not become entangled. It would be further beneficial if the suture package is configured to prevent the barbs from becoming flat or otherwise damaged. Patent document DE 2618662 discloses a device according to the preamble of claim 1.

### SUMMARY

Accordingly, a suture package configured to retain one or more barbed suture is provided. The suture package includes, a base member having an outer wall and a suture retaining area, at least one needle retainer positioned adjacent the suture retaining area for receiving at least one suture needle, at least one suture retainer positioned adjacent the suture retaining area for receiving a distal end of at least one suture, a plurality of raised portions formed within the suture retaining area, wherein each of the raised portions includes a groove for receiving a portion of at least one suture therein.

The groove in each of the raised portions may be formed on an outer wall-facing surface of the raised portion. The groove in each of the raised portions may extend at least partially around the raised portion. The at least one needle retainer may include a foam strip. The at least one suture retainer may include a foam strip. The foam strip may be configured to receive a suture end effector. The raised portions may be disposed at an angle relative to the outer wall of the base member. The suture may be a barbed suture. The suture package may include at least five raised portions. The outer wall of the suture package may include a plurality of inward facing tabs for releasably engaging a cover.

An alternate embodiment of a suture package configured to retain one or more barbed sutures is also provided. The suture package includes a base member having an outer wall and including a suture retaining area, a needle retainer positioned adjacent the suture retaining area for receiving a plurality of suture needles, a plurality of suture retaining means positioned adjacent the suture retaining area, each of the suture retaining means configured for receiving a distal end of a suture, and two or more pairs of longitudinally spaced and laterally offset raised portions formed within the suture retaining area configured to receive a suture thereabout, wherein each of the raised portions include a groove for selectively engaging a suture.

The groove in each of the raised positions may be formed in a longitudinally outward facing surface of the raised portion. The groove in each of the raised portions may extend at least partially about the raised portion. The at least one needle retainer may include a foam strip. The at least one suture retainer may include a foam strip. The outer wall may include a plurality of inward facing tabs for releasably engaging one or more cover members.

### BRIEF DESCRIPTION OF THE DRAWINGS

The accompanying drawings, which are incorporated in and constitute a part of this specification, illustrate embodiments of the disclosure and, together with a general description of the disclosure given above, and the detailed description of the embodiments given below, serve to explain the principles of the disclosure, wherein:

FIG. 1 is a perspective view of a suture package according to an embodiment of the present disclosure, including a base member and a cover member;

FIG. 2 is a top view of the base member of the suture package of FIG. 1;

FIG. 3 is a side view of the base member of FIG. 2;

FIG. 4 is a top view of the base member of FIGS. 2 and 3, including a barbed suture;

FIG. 5 is a top view of a suture package according to an alternate embodiment of the present disclosure;

FIG. 6 is a side view of the suture package of FIG. 5;

FIG. 7 is a cross-section side view of the suture package of FIGS. 5 and 6;

FIG. 8 is an enlarged cross-sectional view of a portion of the suture package of FIGS. 5-7;

FIG. 9 is a perspective of a suture package according to another embodiment of the present disclosure, including a base member and a pair of cover members;

FIG. 10 is a side view of the suture package of FIG. 9;

FIG. 11 is a top view of the suture package of FIGS. 9 and 10;

FIG. 12 is a top view of base member of the suture package of FIGS. 9-11, including a plurality of barbed sutures;

FIG. 13 is a perspective view of a suture package according to yet another embodiment of the present disclosure;

FIG. 14 is a top view of the suture package of FIG. 13;

FIG. 15 is a side view of the suture package of FIG. 14; and

FIG. 16 is an end view of the suture package of FIG. 15.

### DETAILED DESCRIPTION

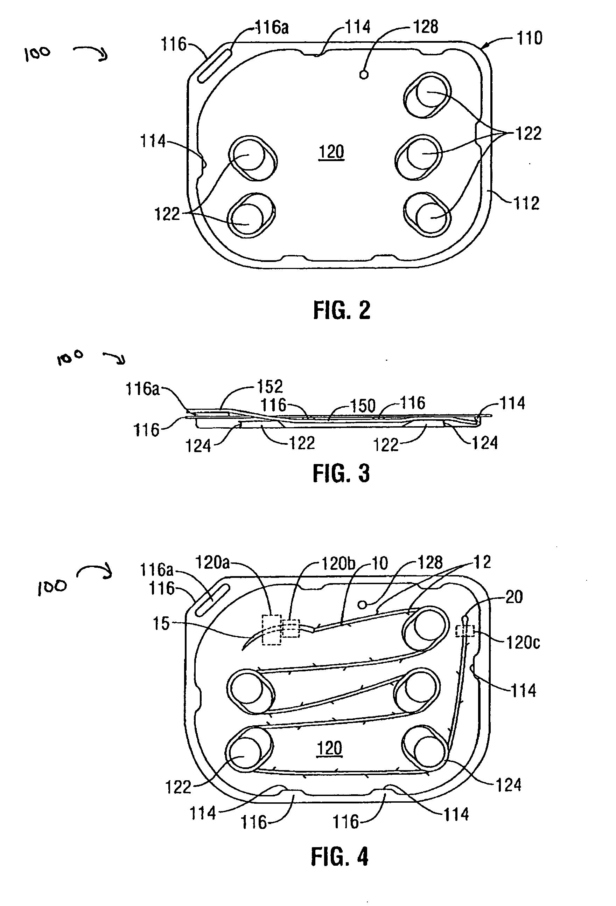

The following disclosure will describe various embodiments of a suture package. Although continued reference will be made to a barbed suture 10 (FIG. 2), having an end effector 20 on a first end, a needle 15 on an opposite end, and barbs 12 formed along the length thereof, it is envisioned that the aspects of the present disclosure may be modified for use with sutures of all sizes and configurations, including several types of needles and end effectors. The disclosure should not be limited to the sutures herein described.

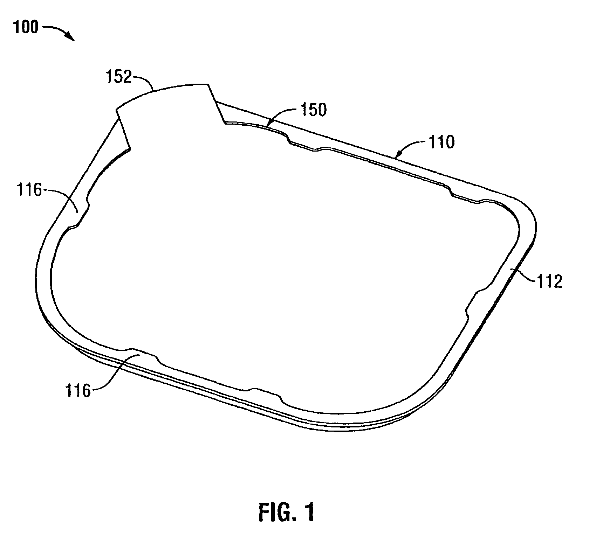

With reference to FIGS. 1-4, a first embodiment of a suture package according to the present disclosure is shown generally as suture package 100. Refening initially to FIG. 1, suture package 100 includes a base member 110 configured for receiving one or more sutures 10 (FIG. 4) and a cover member 150 for retaining one or more sutures 10 within base member 110. As shown, base member 110 includes a substantially rectangular configuration, however, however, alternative configurations have been contemplated, including square, circular and elliptical. As will be discussed in further detail below, cover member 150 includes a substantially similar configuration as base member 110 and is configured to selectively engage base member 110.

Referring now to FIGS. 2-4, base member 110 of suture package 100 is configured to receive barbed suture 10 (FIG. 4). Base member 110 may be formed of plastic, polymer or any other moldable material. Base 110 includes an outer wall 112 defining a suture retaining area 120. Suture retaining area 120 includes a plurality of raised portions 122 formed therein. Raised portions 122 may be integrally formed with base member 110, as shown. In an alternative embodiment, raised portions 122 may be securely affixed within suture retaining portion 120. Although shown as including five (5) raised portions 122, suture retaining portion 120 may include any number of raised portions 122. Raised portions 122 may be of any size or configuration. A wider raised portion 122 requires less flexion of barbed suture 10 as barbed suture 10 is wrapped about raised portion 122. Raised portions 122 may be arranged in any configuration in which barbed suture 10 may be wrapped thereabout without barbs 12 of barbed suture 10 engaging one another. Raised portions 122 include grooves or slot 124 formed in an outwardly facing surface of raised portions 122. Grooves 124 are configured to receive a portion of barbed suture 10 therein. Although, as shown, grooves 124 only extend about an outwardly facing surface of raised portions 122, it is envisioned that grooves 124 may partially or completely circumscribe raised portions 122. It is further envisioned that each of raised portions 122 may include more than one groove 124 for receiving more than one barbed suture 10. In this manner, multiple barbed sutures 10 may be received and maintained separate from one another within suture retaining area 120. Grooves 124 may also be configured to support or suspend barbed suture 10 within suture retaining area 120 such that barbs 12 on barbed suture 10 minimally contact base member 110 and cover member 150. Raised portions 122 are of a sufficient height to support cover member 150 such that a space is created within suture retaining area 120 in which barbed suture 10 may be retained without barbs 12 formed thereon being compressed.

With particular reference to FIG. 4, suture retaining area 120 of base member 110 further includes at least one of a needle park 120a and a first foam pad 120b (both shown in phantom), for selectively receiving suture needle 15. Suture retaining area 120 may also include a second foam pad 120c (also shown in phantom) or other suture retaining means, for selectively retaining a distal end of barbed suture 10. As shown, second foam pad 120c is configured to selectively engage an end of suture 10 including end effector 20. In an alternate embodiment, suture needle 15 and/or end effector 20 of suture 10 may be loosely received with suture retaining area 120 of base member 110. Suture retaining area 120 further includes an aperture or slot 128 configured to selectively receive an alignment pin (not shown) of a loading apparatus (also not shown) for when a loading apparatus is used to load barbed suture 10 within suture retaining area 120.

Turning back to FIGS. 1-3, outer wall 112 of base member 110 includes a plurality of inwardly extending ridges 114 configured to releasably engage cover member 150. Ridges 114 are configured and positioned such that an outer perimeter of cover member 150 engages ridges 114 when cover member 150 is received within outer wall 112. Outer wall 112 of base member 110 further includes a tab region 116 extending outwardly from base member 110. Tab region 116 includes a bump 116a or otherwise raised configuration for permitting engagement of a tab portion 152 of cover member 150 by a clinician. Bump 116a maintains tab portion 152 spaced away from tab region 116, when cover member 150 is selectively engaged with base member 110.

With reference still to FIG. 1, cover member 150 defines a substantially similar shape to base member 110. Cover member 150 may be formed of plastic, paper or any other semi-rigid material. Cover member 150 is configured to be received within suture retaining area 120 of base member 110 and selectively engage ridges 114 extending inwardly from outer wall 112. As discussed above, cover member 150 includes a tab portion 152 corresponding to tab region 116 formed in outer wall 112 of base member 110. Tab portion 152 of cover member 150 is configured for operable engagement by a clinician to permit separation of cover member 150 from base member 110 to expose barbed suture 10 retained within suture retaining area 120.

The use of suture package 100 will now be described with reference to FIGS. 1-4. Barbed suture 10 may be loaded into base member 110 manually or with the use of a loading apparatus. As discussed above, suture retaining area 120 of base member 110 includes an aperture 128 configured for receiving an alignment pin (not show) of a loading apparatus (also not shown). In the event that a loading apparatus is used to load barbed suture 10 within suture package 100, base member 110 is initially loaded onto the loading apparatus by aligning aperture 128 with an alignment pin of the loading apparatus. Next, a first end of barbed suture 10 is loaded within one of needle park 120a, first foam pad 120b or second foam pad 120c, depending on whether the first end of barbed suture 10 includes end effector 20 or suture needle 15. Barbed suture 10 is then wrapped around each of raised portions 122 such that barbed suture 10 is received within grooves 124 formed therein. Once barbed suture 10 has been received about raised portions 122, the second end of barbed suture 10 is selectively received within the other of needle park 120a, first foam pad 120b and second foam pad 120c.

Once barbed suture 10 is securely received within suture retaining area 120, cover member 150 is positioned within suture retaining area 120 such that tab region 116 of base member 110 and tab portion 152 of cover member 150 align. Cover member 150 is then selectively secured to base member 110 by engaging a perimeter of cover member 150 with ridges 114 extending inwardly from outer wall 112. Bump 116a formed on tab region 116 of base member 110 maintains tab portion 152 spaced from outer wall 112 to facilitate engagement by a clinician when access to suture 10 is necessary. Barbed suture 10 may be removed from suture package 100 in a reverse manner to its loaded. Suture package 100 may be hermetically sealed or otherwise packaged for shipping and handling.

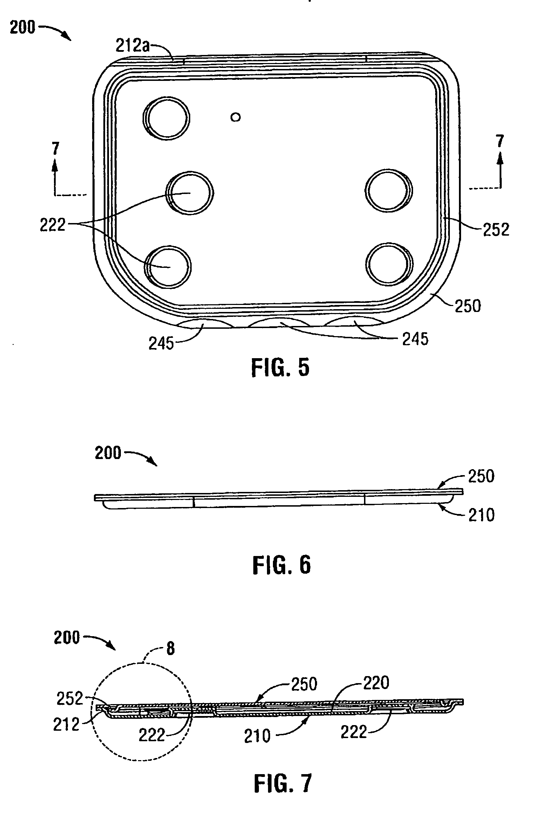

Turning now to FIGS. 5-8, an alternate embodiment of a suture package according to the present disclosure is shown generally as suture package 200. Suture package 200 is substantially similar to suture package 100. Suture package 200 includes a base member 210 and a cover member 250. Base member 210 includes an outer wall 212 defining a suture retaining area 220. Suture retaining area 220 includes a plurality of raised portions 222 configured to selectively retain barbed suture 10 (FIG. 4). Cover member 250 is composed of a rigid plastic and may be clear to permit viewing of the contents thereunder. Cover member 250 is hingedly attached to a first length 212a of outer wall 212. Cover member 250 is configured to be folded along first length 212a and over suture retaining area 220 to selectively retain barbed suture 10 within suture retaining area 220. In one embodiment, cover member 250 contacts raised portions 222 to prevent barbed sutures 10 from disengaging raised portions 222, however, in alternate embodiments cover member 250 may not contact raised portions 222. An outer perimeter 252 of cover member 250 is configured to selectively engage outer wall 212. In one embodiment, outer perimeter 252 engages outer wall 212 in a snap-fit manner (FIG. 5). Outer perimeter 252 may extend completely about cover member 250, as shown, or outer perimeter 252 may extend about only a portion of cover member 250. Barbed suture 10 is loaded and unloaded from suture package 200 in substantially the same manner as barbed suture 10 is loaded and unloaded from suture package 100.

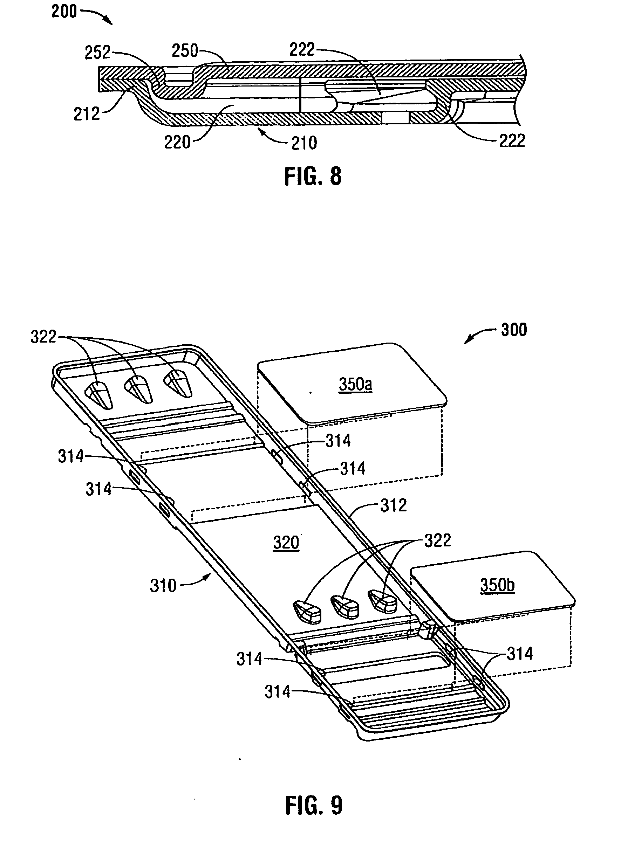

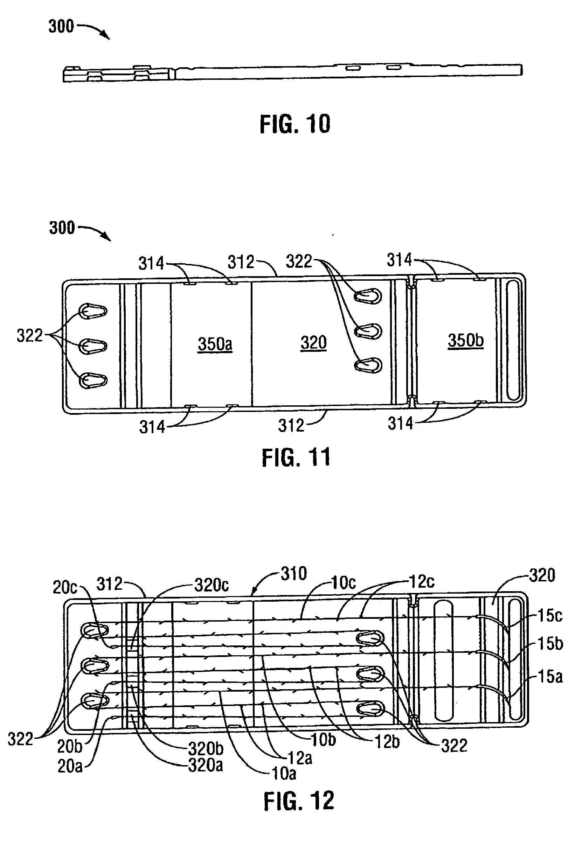

With reference now to FIGS. 9-12, another embodiment of a suture package according to the present disclosure is shown generally as suture package 300. Suture package 300 is substantially similar to suture packages 100, 200 described hereinabove. Suture package 300 includes an elongated base member 310 configured to receive a plurality of sutures 10a-c (FIG. 12). The length of base member 310 determines the length of sutures 10a-c. Sutures 10a-c may be of any size or configuration and may be identical to one another or different. Base member 310 includes an outer wall 312 that defines a suture retaining area 320. A plurality of raised portions 322 are formed in suture retaining area 320 and are configured to selectively engage a plurality of barbed sutures 10 thereabout. Each pair of raised portions 322 are longitudinally spaced and laterally offset from one another such that suture 10a may be wrapped thereabout without the barbs 12a of the suture engaging one another or the barbs 12b of an adjacent suture 10b. Although, as shown, base member 310 includes three (3) pairs of raised portions 322 configured to receive three (3) barbed sutures 10a-c, base member 310 may be configured to with any number (n) of pairs of raised portions 322 to receive any number (n) of barbed sutures 10. A needle retaining foam strip 320a is configured to receive needles 15a-c of sutures 10a-c. A plurality of foam pads 320c are configured to engage a end of respective suture 10a-c including end effector 20a-c. A plurality of ridges 314 extend inwardly from outer wall 312 in a manner similar to ridges 114 described on outer wall 112 of base member 110 of suture package 100 hereinabove. Ridges 314 are configured to selectively engage cover members 350a, 350b.

With particular reference to FIGS. 9 and 11, cover members 350a, 350b may be formed of plastic, paper or any other semi-rigid material. Cover members 350a, 350b are configured to be received within suture retaining area 320 of base member 310 and selectively engage ridges 314 extending inwardly from outer wall 312. Cover members 350a, 350b selectively retain suture 10a-c within suture retaining area 320. Once sutures 10a-c are securely received within suture retaining area 320, suture package 300 may be hermetically sealed in any known manner.

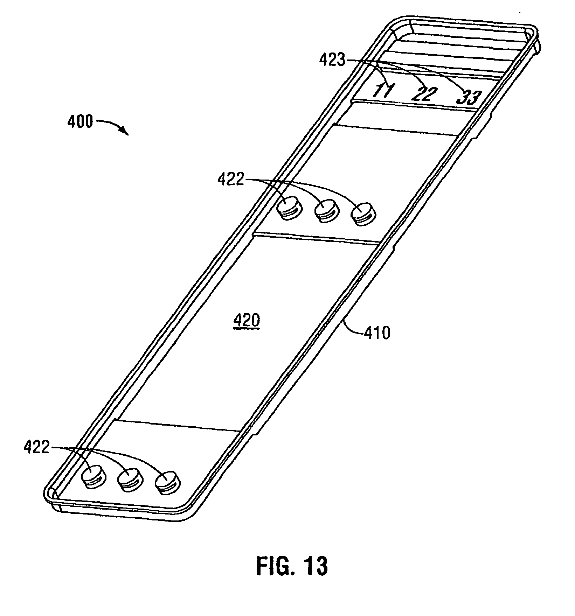

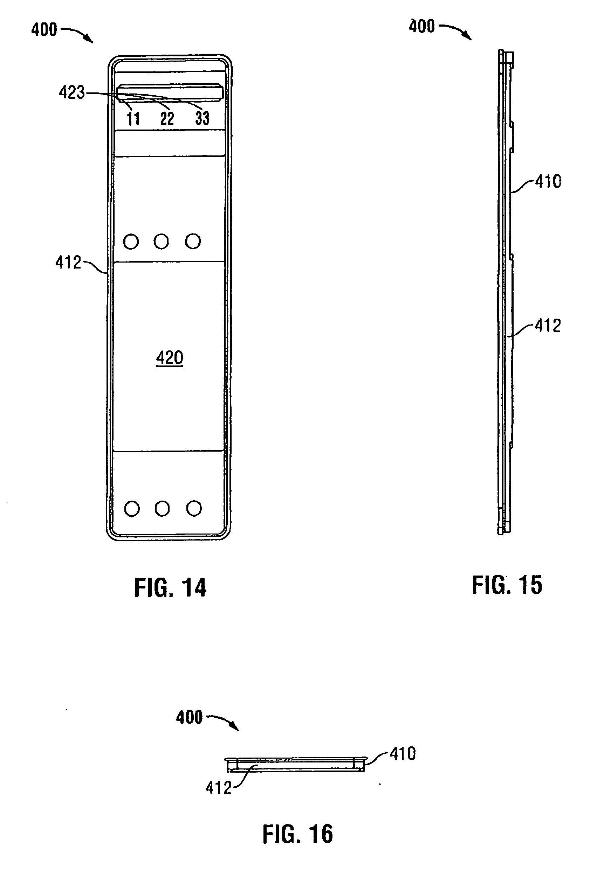

With reference now to FIGS. 13-16, yet another embodiment of a suture package of the present disclosure is shown generally as suture package 400. Suture package 400 is substantially identical to suture package 300 described hereinabove. Suture package 400 includes an elongated base member 410 configured to selectively retain a plurality of sutures (FIG. 12). Base member 410 includes an outer wall 412 that defines a suture retaining area 420. Suture retaining area 420 includes a plurality of pairs of raised portions 422. Suture retaining area 420 further includes reference characters 423 formed thereon in alignment with raised portions 422 that maybe used a clinician to identify the type of suture retained on each of the pairs of raised portions 222. Suture package 400 is loaded in substantially the same manner as each of suture packages 100, 200, 300 described hereinabove.

Although the illustrative embodiments of the present disclosure have been described herein with reference to the accompanying drawings, it is to be understood that the disclosure is not limited to those precise embodiments, and that various other changes and modifications may be effected therein by one skilled in the art without departing from the scope of the disclosure.

## Claims

1. A suture package (100) comprising:
a base member (110) having an outer wall (112) and including a suture retaining area (120);
at least one needle retainer positioned adjacent the suture retaining area (120) for receiving at least one suture needle (15);
at least one suture retainer positioned adjacent the suture retaining area (120) for receiving a distal end of at least one suture (10);
a plurality of raised portions (122) formed within the suture retaining area (120), each of the raised portions (122) including more than one groove (124) for receiving a portion of at least one suture (10) therein; **characterised in that** it comprises
a cover (150); and **in that**
the outer wall (112) includes a plurality of inward facing tabs (114) for releasably engaging the cover (150).

2. The suture package (100) of claim 1, wherein the groove (124) in each of the raised portions (122) is formed on an outer wall-facing surface of the raised portion (122).

3. The suture package (100) of claim 1 or claim 2, wherein the groove (124) in each of the raised portions (122) extends at least partially around the raised portion (122).

4. The suture package (100) of any preceding claim, wherein the at least one needle retainer includes a foam strip (120b).

5. The suture package (100) of any preceding claim, wherein the at least one suture retainer includes a foam strip (120c).

6. The suture package (100) of claim 5, wherein the foam strip (120c) is configured to receive a suture end effector (20).

7. The suture package (100) of any preceding claim, wherein the raised portions (122) are disposed at an angle relative to the outer wall (112) of the base member (110).

8. The suture package (100) of any preceding claim, wherein the suture (10) is a barbed suture.

9. The suture package (100) of any preceding claim, including at least five (5) raised portions (122).

10. A suture package (300) comprising:
a base member (310) having an outer wall (312) and including a suture retaining area (320);
a needle retainer positioned adjacent the suture retaining area (320) for receiving a plurality of suture needles (15a, 15b, 15c);
a plurality of suture retaining means positioned adjacent the suture retaining area (320), each of the suture retaining means configured for receiving a distal end of a suture (10a, 10b, 10c); and
two or more pairs of longitudinally spaced and laterally offset raised portions (322) formed within the suture retaining area (320) configured to receive a suture (10a, 10b, 10c) thereabout, wherein each of the raised portions (322) include a groove for selectively engaging a suture (10a, 10b, 10c); **characterised in that** it comprises
one or more cover members (350a, 350b); and **in that**
the outer wall (312) includes a plurality of inward facing tabs (314) for releasably engaging the one or more cover members (350a, 350b).

11. The suture package (300) of claim 10, wherein the groove in each of the raised portions (322) is formed in a longitudinally outward facing surface of the raised portion (322).

12. The suture package (300) of claim 10 or claim 11, wherein the groove in each of the raised portions (322) extends at least partially about the raised portion (322).

13. The suture package (300) of any of claims 10 to 12, wherein the at least one needle retainer includes a foam strip.

14. The suture package of any of claims 10 to 13, wherein the at least one suture retainer includes a foam strip (320a, 320b, 320c).

## Patentansprüche

1. Nahtmaterialpackung (100), die Folgendes umfasst:
eine Trägerplatte (110), die eine äußere Wand (112) hat und einen Nahthaltebereich (120) beinhaltet,
mindestens einen Nadelhalter, der neben dem Nahthaltebereich (120) zur Aufnahme von mindestens einer Nahtnadel (15) positioniert ist,
mindestens einen Nahthalter, der neben dem Nahthaltebereich (120) zur Aufnahme eines distalen Endes von mindestens einem Nahtfaden (10) positioniert ist,
eine Vielzahl von angehobenen Abschnitten (122), die innerhalb des Nahthaltebereichs (120) gebildet wurden, wobei jeder der angehobenen Abschnitte (122) mehr als eine Rille (124) zur Aufnahme eines Abschnitts von mindestens einem Nahtfaden (10) darin beinhaltet, **dadurch gekennzeichnet, dass** es eine Abdeckung (150) umfasst,
und dass die äußere Wand (112) eine Vielzahl von nach innen zeigenden Laschen (114) zum lösbaren Einrasten der Abdeckung (150) beinhaltet.

2. Nahtmaterialpackung (100) nach Anspruch 1, wobei die Rille (124) in jedem der angehobenen Abschnitte (122) an einer der äußeren Wand zugewandten Oberfläche des angehobenen Abschnitts (122) gebildet wird.

3. Nahtmaterialpackung (100) nach Anspruch 1 oder 2, wobei sich die Rille (124) in jedem der angehobenen Abschnitte (122) mindestens teilweise um den angehobenen Abschnitt (122) erstreckt.

4. Nahtmaterialpackung (100) nach einem vorangehenden Anspruch, wobei der mindestens eine Nadelhalter einen Schaumstreifen (120b) beinhaltet.

5. Nahtmaterialpackung (100) nach einem vorangehenden Anspruch, wobei der mindestens eine Nahthalter einen Schaumstreifen (120c) beinhaltet.

6. Nahtmaterialpackung (100) nach Anspruch 5, wobei der Schaumstreifen (120c) so konfiguriert wurde, dass er einen Effektor für das Nahtfadenende aufnimmt (20).

7. Nahtmaterialpackung (100) nach einem vorangehenden Anspruch, wobei die angehobenen Abschnitte (122) in einem Winkel relativ zur äußeren Wand (112) der Trägerplatte (110) angeordnet sind.

8. Nahtmaterialpackung (100) nach einem vorangehenden Anspruch, wobei der Nahtfaden (10) ein chirurgischer Faden mit Widerhaken ist.

9. Nahtmaterialpackung (100) nach einem vorangehenden Anspruch, die mindestens fünf (5) angehobene Abschnitte (122) beinhaltet.

10. Nahtmaterialpackung (300), die Folgendes umfasst:
eine Trägerplatte (310), die eine äußere Wand (312) hat und einen Nahthaltebereich (320) beinhaltet,
einen Nadelhalter, der neben dem Nahthaltebereich (320) zur Aufnahme einer Vielzahl von Nahtnadeln (15a, 15b, 15c) positioniert ist.
eine Vielzahl von Nahthaltemitteln, die neben dem Nahthaltebereich (320) positioniert sind, wobei jedes der Nahthaltemittel zur Aufnahme eines distalen Endes eines Nahtfadens (10a, 10b, 10c) konfiguriert ist, und
zwei oder mehrere Paare von der Länge nach im Abstand angeordneten und lateral versetzten angehobenen Abschnitten (322), die innerhalb des Nahthaltebereichs (320) gebildet sind und zur Aufnahme eines Nahtfadens (10a, 10b, 10c) konfiguriert wurden, wobei jeder der angehobenen Abschnitte (322) eine Rille zum selektiven Einrasten eines Nahtfadens (10a, 10b, 10c) beinhaltet, **dadurch gekennzeichnet, dass** er ein oder mehrere Abdeckungselemente (350a, 350b) umfasst,
und dass die äußere Wand (312) eine Vielzahl von nach innen gerichteten Laschen (314) zum lösbaren Einrasten der einen oder mehreren Abdeckungselemente (350a, 350b) beinhaltet.

11. Nahtmaterialpackung (300) nach Anspruch 10, wobei die Rille in jedem der angehobenen Abschnitte (322) in einer der Länge nach außen zeigenden Oberfläche des angehobenen Abschnitts (322) gebildet wurde.

12. Nahtmaterialpackung (300) nach Anspruch 10 oder 11, wobei sich die Rille in jedem der angehobenen Abschnitte (322) mindestens teilweise um den angehobenen Abschnitt (322) erstreckt.

13. Nahtmaterialpackung (300) nach einem der Ansprüche 10 bis 12, wobei der mindestens eine Nadelhalter einen Schaumstreifen beinhaltet.

14. Nahtmaterialpackung nach einem der Ansprüche 10 bis 13, wobei der mindestens eine Nadelhalter einen Schaumstreifen (320a, 320b, 320c) beinhaltet.

## Revendications

1. Emballage de suture (100) comprenant :
un élément de base (110) ayant une paroi externe (112) et comprenant une surface de retenue de suture (120) ;
au moins un élément de retenue d'aiguille positionné adjacent à la surface de retenue de suture (120) pour recevoir au moins une aiguille de suture (15) ;
au moins un élément de retenue de suture positionné adjacent à la surface de retenue de suture (120) pour recevoir une extrémité distale d'au moins une suture (10) ;
une pluralité de portions tressées (122) formées dans la surface de retenue de suture (120), chacune des portions dressées (122) comprenant plus d'une rainure (124) pour y recevoir une portion d'au moins une suture (10) ; **caractérisé en ce qu'**il comprend :
une coiffe (150) ; et **en ce que**
la paroi externe (112) comprend une pluralité de pattes (114) tournées vers l'intérieur pour s'engager sur la coiffe (150) de manière libérable.

2. Emballage de suture (100) selon la revendication 1, dans lequel la rainure (124) de chacune des portions dressées (122) est formée sur une surface de la portion dressée (122) tournée vers la paroi externe.

3. Emballage de suture (100) selon la revendication 1 ou la revendication 2, dans lequel la rainure (124) de chacune des portions dressées (122) s'étend au moins en partie autour de la portion dressée (122).

4. Emballage de suture (100) selon l'une quelconque des revendications précédentes, dans lequel le au moins un élément de retenue d'aiguille comprend une bande de mousse (120b).

5. Emballage de suture (100) selon l'une quelconque des revendications précédentes, dans lequel le au moins un élément de retenue de suture comprend une bande de mousse (120c).

6. Emballage de suture (100) selon la revendication 5, dans lequel la bande de mousse (120c) est configurée pour recevoir un effecteur d'extrémité de suture (20).

7. Emballage de suture (100) selon l'une quelconque des revendications précédentes, dans lequel les portions dressées (122) sont disposées selon un certain angle par rapport à la paroi externe (112) de l'élément de base (110).

8. Emballage de suture (100) selon l'une quelconque des revendications précédentes, dans lequel la suture (10) est une suture à crans.

9. Emballage de suture (100) selon l'une quelconque des revendications précédentes, comprenant au moins cinq portions dressées (122).

10. Emballage de suture (300) comprenant :
un élément de base (310) ayant une paroi externe (312) et comprenant une surface de retenue de suture (320) ;
un élément de retenue d'aiguille positionné adjacent à la surface de retenue de suture (320) pour recevoir une pluralité d'aiguilles de suture (15a, 15b, 15c) ;
une pluralité de moyens de retenue de suture positionnés adjacents à la surface de retenue de suture (320), chacun des moyens de retenue de suture étant configuré pour recevoir une extrémité distale d'une suture (10a, 10b, 10c) ; et
deux paires ou plus de portions dressées (322) espacées longitudinalement et décalées latéralement, formées dans la surface de retenue de suture (320), configurées pour y recevoir une suture (10a, 10b, 10c), dans lequel chacune des portions dressées (322) comprend une rainure pour s'engager sélectivement sur une suture (10a, 10b, 10c) ; **caractérisé en ce qu'**il comprend :
un ou plusieurs éléments de coiffe (350a, 350b) ; et **en ce que**
la paroi externe (312) comprend une pluralité de pattes (314) tournées vers l'intérieur pour s'engager de manière libérable sur le ou les éléments de coiffe (350a, 350b).

11. Emballage de suture (300) selon la revendication 10, dans lequel la rainure de chacune des portions dressées (322) est formée dans une surface, tournée longitudinalement vers l'extérieur, de la portion dressée (322).

12. Emballage de suture (300) selon la revendication 10 ou la revendication 11, dans lequel la rainure de chacune des portions dressées (322) s'étend au moins en partie autour de la portion dressée (322).

13. Emballage de suture (300) selon l'une quelconque des revendications 10 à 12, dans lequel le au moins un élément de retenue d'aiguille comprend une bande de mousse.

14. Emballage de suture selon l'une quelconque des revendications 10 à 13, dans lequel le au moins un élément de retenue de suture comprend une bande de mousse (320a, 320b, 320c).
